# EUROPEAN PATENT SPECIFICATION

(11) **EP 3 430 923 B1**
(45) Date of publication and mention of the grant of the patent: **21.10.2020**
(21) Application number: 18183425.0
(22) Date of filing: 13.07.2018
(51) Int. Cl.: A41B 11/00, A61F 13/08, A61F 13/06

(54) **MEDICAL SOCK**
MEDIZINISCHE STRUMPFE
CHAUSSETTE MÉDICALE

(30) Priority: 20.07.2017 BE 201705516
(43) Date of publication of application: 23.01.2019
(73) Proprietor: DeCo-Vision BVBA, 9800 Deinze (BE)
(72) Inventor: De Coninck, Johan, 9800 Deinze (BE)
(74) Representative: Brantsandpatents bvba

(56) References cited:
- EP-A2- 1 079 017
- WO-A1-2012/058708
- US-B1- 8 220 077

## Description

### TECHNICAL DOMAIN

The invention relates to an improved medical sock, more in particular a compression sock or support sock for preventing blood clots from forming in the lower limbs of a person wearing them.

### STATE OF THE ART

Support socks or medical compressions socks, also known as therapeutic or anti-embolic socks, create a compressive pressure on a leg and/or foot of a person wearing them. The socks are typically worn by persons whose mobility is limited, for example after surgery, during long-term illness, during long flights, or other situations in which long immobility (or reduced mobility) occurs. Furthermore, in more daily situations, they are also worn by seniors, or other persons with reduced mobility. Reduced mobility can cause a reduction in the speed with which blood flows in the legs of a user, which can result in an increased risk of thrombosis or emboly. By exerting a pressure onto the leg and/or foot, the speed with which the blood flows, is increased and thus these risks can be minimized. In general, the socks exert a pressure onto the legs (and/or foot) according to a pressure gradient that decreases with the height of the sock (higher pressure at the ankle, lower pressure above it)

Such medical compression socks are mostly worn without any shoes, leading to an increased risk for the user of slipping. This is even more true for hospital patients, who often wear compression socks without any shoes, who are weakened, tired, disabled and/or who are taking medication, and thus have an increased risk of slipping. Moreover, it should be remarked that hospital floors are typically strongly polished. Therefore, some hospitals already provide patients and staff with non-slip shoe coverings. However, they are quite expensive and are often not worn by the patients, and it is also difficult to oblige patients at all time to wear them.

WO 2012/058708 discloses a compression sock according to the preamble of claim 1.

Prior art such as in US 4,021,860 shows a compression sock with anti-slip elements, by attaching a thermoplastic material to the sole of the compression sock. However, it has a number of disadvantageous aspects that one the one hand make the use of the sock impractical, and on the other hand make the operation inefficient. The thermoplastic material is not sufficiently elastic to completely allow deformation of the sock and can thus cause discomfort with the user. The most important disadvantageous effect is however that, as a result of this incomplete deformation of the sock due to the rigid gripping elements, the compressive effect of the sock is strongly influenced (locally), since the sock cannot stretch equally everywhere and thus will exert other pressures on the leg or on the foot of the user, depending on the presence or absence or even vicinity of a gripping element at these positions.

A problem with the known socks is that they are not sufficiently adapted to meet the specific needs of this situation. Because of an inappropriate choice of material and/or configuration of the anti-slip elements, they cannot offer a good grip because of an insufficiently increased friction coefficient between the compression sock and the (often slippery) floor.

The present invention aims to find a solution for at least some of the above-mentioned problems.

### SUMMARY OF THE INVENTION

The invention relates to an improved compression sock according to claim 1.

All gripping elements have a minimal surface of about 0,2 cm² and a maximal surface of about 1 cm², preferably a minimal surface of about 0,225 cm² and/or a maximal surface of about 0,50 cm², more preferably a maximal surface of about 0,40 cm², still more preferably a maximal surface of 0,35 cm² and most preferably a maximal surface of 0,30 cm².

### DESCRIPTION OF THE FIGURES

**Figure 1A-B** shows a first embodiment of the compression sock according to the invention in a side view and a lower view.
**Figure 2A-B** shows a second embodiment of the compression sock according to the invention in a side view and a lower view.
**Figure 3A-B** shows a third embodiment of the compression sock according to the invention in a side view and a lower view.
**Figure 4A-B** shows a fourth embodiment of the compression sock according to the invention in a side view and a lower view.
**Figure 5A-B** shows a fifth embodiment of the compression sock according to the invention in a side view and a lower view.
**Figure 6A-B** shows a sixth embodiment of the compression sock according to the invention in a side view and a lower view.
**Figure 7A-B** shows a seventh embodiment of the compression sock according to the invention in a side view and a lower view.
**Figure 8A-B** shows an eight embodiment of the compression sock according to the invention in a side view and a lower view.

### DETAILED DESCRIPTION

The invention relates to an improved compression sock for preventing blood clots from forming, in which an improved slipping resistance is created. The applicant has noted that the use of compression socks, especially in hospitals, entails some dangers. In such settings, most users of compression socks don't wear shoes, and moreover, the floors are slippery. This combined with an already weakened user (for example as a result of an illness), leads to many unnecessary accidents. Therefore, the applicant has developed a compression sock with an optimized anti-slip effect, which will be discussed later in a number of more detailed aspects.

Unless otherwise specified, all terms used in the description of the invention, including technical and scientific terms, shall have the meaning as they are generally understood by the worker in the technical field of the invention. For a better understanding of the description of the invention, the following terms are explained specifically.

"A", "an" and "the" refer in the document to both the singular and the plural form unless clearly understood differently in the context. "A segment" means for example one or more than one segment.

The term "foot part" refers to a preformed part of the compression sock that is appropriate for comprising a foot of a user. Typically, the foot part also comprises a specifically adapted heel part, for positioning the compression sock correctly with respect to the heel and for preventing shifting, and moreover also a toe part that is preformed for receiving toes of a user. In a side view, the foot part is typically in an angle with respect to the leg part to follow at least partially the natural angle of a foot with respect to a leg.

The term "leg part" refers to a preformed part of the compression sock that is appropriate for comprising a leg of a user. Typically, the leg part is approximately cylindrical or truncated conical, or a combination of several such shapes, in order to take the shape of a human leg when it is present therein, and thereby to create the desired pressure in the leg. The leg part typically extends from the heel till under the knee or till above the knee or even till or over the thigh.

The term "longitudinal axis of the foot part" refers to a virtual axis that extends approximately from heel to toe (or heel part to toe part of the foot part), specifically the middle toe or a central point of the toes at the user when the compression sock is worn by the user.

The term "transversal" refers to a sideward direction in the plane of the foot (sole) at both sides of the longitudinal axis of the foot part as described above. The "transversal width" is thus the width along this direction.

The term "calf zone" refers to a part of the leg described as approximately the thickest (largest circumference) part of the leg. Furthermore, this zone also deviates from the rest of the leg because a calf is typically wider and has a truncated conical (or approximately conical) shape, according to the widening calf.

The term "upper ankle zone" refers to a part of the leg that is the thinnest (smallest circumference) part of the leg.

The term "stiches" refers to the filaments of the woven material.

When "approximately" or "about" are used in the document together with a measurable quantity, a parameter, a period or moment, etc., variations of +/-20% or less, preferably +/-10% or less, more preferably +/-5% or less, still more preferably +/-1% or less, and even still more preferably +/-0.1% or less than and of the cited value are meant, as far as such variations apply to the invention that is described. It will however be clearly understood that the value of the quantity at which the term "approximately" or "about" is used, is itself specified.

The terms "include", "including", "consist", "consisting", "provide with", "contain", "containing", "comprise", "comprising" are synonyms and are inclusive of open terms that indicate the presence of what follows, and that do not exclude or prevent the presence of other components, characteristics, elements, members, steps, known from or described in the state of the art.

The citation of numeric intervals by means of end points includes all integers, fractions and/or real numbers between the end points, including these end points.

In a first aspect, the invention relates to a compression sock for preventing blood clots from forming. The compression sock comprises an elastic foot part that is appropriate for receiving a foot of a user, and an elastic leg part that is appropriate for receiving at least a part of a leg of the user. The part of the leg that is received by the leg part is at least the ankle and the knee, and optionally the thigh of the user. The foot part comprises an external bottom side that is appropriate for contacting the foot with the floor. Hereby, the external bottom side of the foot part is provided with a plurality of gripping elements that are adapted for increasing the friction coefficient between the bottom side and the floor, in which the gripping elements each have a minimal surface of about 0,2 cm² and a maximal surface of about 1 cm².

The applicant has noted that many, if not all, of the already known compression socks did few or nothing at all to deal with the safety risks that have been described above. Users of the socks are usually already weakened by illnesses and physical disorders, or they are recovering thereof, and are thus very sensitive for slipping or losing their balance. The compression socks are made of very smooth material, that causes, combined with the very slippery floors of hospitals, rest homes and other rehabilitation centres, many victims of slippings every year. By adding very specific gripping elements to the bottom side of the compression sock, the applicant aims to reduce or even eliminate these problems. The gripping elements are adapted for increasing the friction coefficient between the floor and the compression sock in order to offer a better grip. However, it is important that these gripping elements feel natural, and especially do not make the compression sock itself uncomfortable. Thus, it is for example possible to provide the complete bottom side of the compression sock with a layer of material that offers a better grip, however, this does not feel comfortable for most of the users. Moreover, such a layer would prevent deformation of the compression sock which is crucial for taking the correct shape, namely that of the foot of the user. The compression sock according to the invention is specifically adapted for maximizing comfort and safety. In particular, it is however important that the compression socks can still be stretched out sufficiently despite the presence of the gripping elements, in order to create the desired pressure in the limbs of the person wearing the socks.

Preferably, the gripping elements each have a minimal surface of approximately 0.225 cm² and/or a maximal surface of approximately 0.50 cm².

More preferably, the gripping elements have a maximum surface of approximately 0.40 cm², still more preferably a maximal surface of 0.35 cm² and most preferably, a maximal surface of 0.30 cm².

These further narrowed surfaces offer an optimal configuration of the invention. The applicant has noted that it was more efficient to provide a large number of smaller gripping elements. On the one hand, this maximizes the friction coefficient related to maintaining flexibility of the foot part of the compression sock. Moreover, it has been noticed that at a constant total surface of the gripping elements, it was advantageous to give them a separate surface of maximal 0.30 cm² and thus to provide a larger number of gripping elements. The smaller gripping elements offer a larger increase in friction coefficient per surface unit than larger gripping elements. It should be noticed here that the applicant has already noticed this tendency at surfaces smaller than about 1 cm², but that they were more pronounced at surfaces smaller than about 0.50 cm², 0.40 cm², 0.35 cm², 0.30 cm², but that this tendency reversed under about 0.2 cm² or 0.225 cm². At larger separate surfaces than 1 cm², the gripping elements partially opposed one selves, translating into an inefficient friction coefficient.

In a preferred embodiment, the gripping elements are arranged in a staggered pattern. This pattern is the most efficient 'staple' method in 2 dimensions and allows to position a large number of gripping elements on a limited surface, without placing the separate gripping elements too close one to the other, and without them interfering in a negative way. The applicant has noted that an intermediate distance of at least 5 mm (minimal distance between an edge of a first gripping element and an edge of a second gripping element) is desired, preferably at least 6 mm, and more preferably at least 7 mm, most preferably with a mutual distance of about 7.5 mm or 8 mm.
Conversely, an upper limit of the intermediate distance (again referring to the minimal distance between an edge of a first gripping element and an edge of a second gripping element) has been determined, namely at most 12 mm, in order to be able to place a sufficiently large number of gripping elements, and thus to maximize the total grip. Preferably, the intermediate distance is maximum 10 mm, and more preferably even maximum 9 mm, and still more preferably maximum 8 mm.

Preferably, the gripping elements are placed in a pattern with longitudinal lines onto which the gripping elements are positioned, in which the longitudinal lines extend from heel end to toe end of the foot, and with lateral lines, in which the lateral lines extend over the width of the foot. In this sense, the staggered pattern was thus preferred, in which gripping elements of adjacent longitudinal lines are positioned in a staggered way with respect to each other (see also for example 8A-8B, but this can also be applied to gripping elements with another shape). The applicant has noted that the alignment of the gripping elements leads to an improved total grip, as they work together in a synergistic way along a line, resulting in a larger effect than a scattered spreading of the gripping elements.

Preferably, the gripping elements are arranged in a pattern in which the distance between adjacent gripping elements along an axis substantially along the foot (from heel to toe) is larger than the distance between two adjacent points along line extending laterally over the foot. The applicant has first noted that lateral slipping is a larger problem than 'longitudinal' slipping for socks with gripping elements. Thereby, as described above, the applicant has noticed that an alignment of the gripping elements led to a larger total traction then other spreadings. Moreover, the applicant has found that the closer stapling in the width was necessary to guarantee the necessary traction in the lateral direction, as in this way, 'more' gripping elements could be placed on a lateral line than at an equal distance as along the foot. In this way, a sufficient grip can be ensured over each lateral line, despite the restriction due to the width of the foot.

Preferably, the longitudinal distance between the gripping elements on one and the same longitudinal line is comprised between 8 mm and 22 mm, more preferably between 10 mm and 20 mm, still more preferably between 12 mm and 18 mm, still more preferably between 13 mm and 17 mm, or even between 14 mm and 16 mm, and is most preferably about 15 mm.
The lateral distance between the gripping elements on one and the same lateral line is comprised between 5 mm and 15 mm, preferably between 6 mm and 14 mm, still more preferably between 7 mm and 13 mm, still more preferably between 8 mm and 12 mm, or even between 9 mm and 11 mm, and is most preferably about 10 mm.

In a possible embodiment, the gripping elements are arranged with a larger density (number of gripping elements per surface unity of the compression sock and/or larger surface of gripping elements per surface unity of the compression sock) in certain zones. Preferably, these zones with a larger density are located at the position where the ball of the foot and/or the heel of the foot is located in the compression sock. At these points, the largest pressure/force is exerted, especially when walking (when the risk of slipping is the largest), as a result of which the gripping elements in these zones will be more efficient (as the resulting friction is proportional to the exerted force).

In a preferred embodiment, the gripping elements are adapted to be transversally stretchable with respect to a longitudinal axis of the foot part of the compression sock at least up to 110%, preferably at least up to 120% of a transversal width of the non-stretched gripping elements. More preferably, these are elastic to at least 125% or even 130%, 135%, 140%, 150%, 175% or 200% or more, of the width.

The applicant has noted that with existing compression socks, the gripping elements are (as good as) not deformable, which is pernicious for the comfort of the user. This also causes zones with increased pressure on the sole of the foot of a user which can block blood circulation, but in general can also be disadvantageous for the comfort of the user, who experiences this as rigid, hard points in the sock.
By making the gripping elements slightly deformable at least transversally to the longitudinal axis of the foot, this problem is already partially solved, and from said stretches, to the extent that the user experiences a strongly increased comfort compared to compression socks with (almost) non-deformable gripping elements. As an alternative, rigid gripping elements can be applied in order to compensate for a later stretching of the compression sock, but it is very difficult to position the gripping elements correctly, since this stretching is difficult to provide for. Moreover, this will cause an undesired influence on the compression sock in a non-stretched situation, and this can also cause a deformation of the foot part of the compression sock, influencing the objective of the compression sock - appropriate compression of the foot and the leg of a user - in a negative way. Hereby, it should also be remarked that only stretchability in a transverse direction has been discussed because the compression sock will stretch and deform the most in this direction. However, the gripping elements can preferably also be stretched in a similar way along the longitudinal axis and can thus preferably be stretched at least to 110% of the original dimension along the longitudinal axis of the foot part, more preferably to 120%, 125%, 130%, 135%, 140%, 150%, 175% or 200% or more.

In a preferred embodiment, the leg part comprises an enlarged calf zone, positioned for and appropriate for receiving a calf of the user, and the leg part comprises a narrowed upper ankle zone, positioned for and appropriate for receiving a part of the leg above the ankle of the user. Hereby, the leg part comprises a woven material, characterized in that the woven material comprises more stitches at the level of and around the calf zone than the woven material at the level of and around the upper ankle zone.

In a preferred embodiment, the compression sock comprises at least over a part (preferably at least 20%, more preferably at least 30%, 40%, 50% or more, still more preferably at least 60%, 70% or 80%) of the bottom side of the foot part, exclusive of the gripping elements, a material with increased friction coefficient compared to another part of the compression sock. Most preferably, approximately the complete part of the foot part that is in contact with the floor comprises a material with an increased friction coefficient compared to at least another part of the compression sock, such as for example the upper side of the foot part, the ankle part, etc. The advantage thereof is that it has a further reinforcing effect on the already improved grip on the floor thanks to the gripping elements.
In a possible embodiment, also the upper side of the foot part (completely or partially) comprises the material with increased friction coefficient, as this simplifies the product.
Such a material can for example be created by providing an elastomer yarn in the material, such as for example spandex and/or others.
Typically, such a material also simplifies the attachment of the gripping elements (via heating or compression thereof) onto the compression sock.

The compression sock of the applicant is provided with a 'pocket weaving' at the leg part, in order to follow the shape of the leg as closely as possible and to exert a correct pressure thereon. Prior art compression socks with woven material in the leg part are mostly made via 'cylinder weaving', in which an equal number of stitches is used around the complete length of the leg part. This however does not compensate the strongly variable thickness of the leg of a user. Pocket weaving means that the leg part is produced with a varying stitching density along the length of the leg part. Where the leg is wider (larger circumference, for example a calf), more stitches are used along the circumference of the leg part, then where the leg is smaller (smaller circumference, for example just above the ankle). In this way, the zones with a larger stitching density will stretch further under the same force, allowing to receive the leg of the user in a comfortable way. Note that the stitching density is further configured to exert the appropriate pressure along the length of the leg part, which will be discussed further in this document.

In a preferred embodiment, the gripping elements have a largest dimension on the external bottom side of maximum about 0.75 cm. Preferably, this longest dimension is maximum approximately 0.74 cm, 0.73 cm, or 0.72 cm, or more preferably maximum approximately 0.71 cm, 0.70 cm, 0.69 cm, still more preferably maximum approximately 0.68 cm, 0.67 cm, 0.66 cm, 0.65 cm or even less, for example maximum 0.64 cm, 0.63 cm, 0.62 cm, 0.61 cm, 0.60 cm or still less.

The maximal extent of the largest or longest dimension on the one hand limits the surface of the gripping elements, which has, as discussed above, a positive effect on the friction coefficient. Moreover, the largest dimension is also decisive for the effect that the difference in stretchability can have with respect to the comfort of the user. Below, it had already been indicated that rigid (inelastic) gripping elements are disadvantageous since they do not (or barely) follow the stretching of the foot part of the compression sock, which causes local pressure points, and twisted material at the foot part. The applicant wishes to remark that 'longer' gripping elements (with a larger maximal dimension, the length), are more difficult to deform than shorter ones. Moreover, such longer gripping elements also cause a stronger contrast in stretching with the material of the foot part itself, since the difference in stretching increases with the length of the gripping element. By limiting the longest or largest dimension, the applicant can offer a compression sock in which the undesired consequences of this difference in stretchability are minimized by the user.

In a preferred embodiment, the gripping elements have a largest dimension on the external bottom side of minimal about 0.5 cm. Preferably, this largest dimension is minimum approximately 0.51 cm, 0.52 cm, or 0.53 cm, or more preferably approximately 0.54 cm, 0.55 cm, 0.56 cm, still more preferably 0.57 cm, 0.58 cm, 0.59 cm, 0.6 cm or even more, for example minimum 0.61 cm, 0.62 cm, 0.63 cm, 0.64 cm, 0.65 cm or still more.

The applicant remarks herein that an optimized range can be chosen with an upper limit and lower limit from the two said lists that can depend on a number of variable factors, such as material of the foot part itself and/or material of the gripping element (causing a variable difference in stretchability). However, the lower limit is important to ensure a minimal total surface for the gripping elements to sufficiently increase the friction coefficient. As said above, too little gripping elements cause a less efficient effect on the friction coefficient.

In a preferred embodiment, the leg part is adapted for exerting a pressure onto the leg of the user, in which the pressure that is exerted on the leg part in a zone closer to the foot part is higher than the pressure that is exerted on the leg part in a zone further from the foot part.

The applicant has noted that a specific pressure profile of the leg part on the leg received therein is necessary for preventing blood clots from forming, in which the pressure profile is such that the exerted pressure in a point of the leg part is higher as the point is closer to the foot part.

Preferably, the pressure at the ankle of the user is about 18 - 20 mmHg, and at the knee of the user about 12 - 14 mmHg, and optionally the pressure at the thigh of the user is about 6 - 8 mmHg.

The above values indicate an optimized pressure profile. Please notice however that the pressure profile is preferably continuous, and more preferably shows a smooth function for the exerted pressure with respect to the position of the virtual longitudinal axis along the leg part.

In a preferred embodiment, the foot part comprises a specific heel part, in which the heel part is preformed and is appropriate for gripping and holding in position of the heel of the user, in which the foot part comprises a specific toe part, in which the toe part is preformed anatomically and is appropriate for receiving toes of the user, characterized in that the gripping elements extend at the bottom side at least of the heel part up to the toe part.

With existing compression socks, it is noted that the gripping elements do not fully extend to the end of the heel part, and moreover often also not to the end of the toe part. However, the heel is a crucial point for the grip on a floor, since a very large part of the weight rests thereon, making the possible counterforce as a result of the friction also proportionally larger. By providing the heel part as completely as possible with gripping elements, the counterforce is maximized. The same remark applies to the toe part. Although a smaller part of the weight rests thereon when standing, this is a crucial position when walking, as a person first touches a floor with either the heel or the toes and thus should obtain a stable position as fast as possible. By providing the toe part as completely as possible with gripping elements, the friction coefficient is increased, and falls can be avoided.
Moreover, the provision of a specific heel part ensures that the socks cannot slide off (or slide off less) from the foot of the person wearing them. Moreover, this ensures that the position where maximal compression or pressure is exerted on the leg, can be positioned correctly.
In a preferred embodiment, the gripping elements further protrude from the extern bottom side, in which the gripping elements comprise silicone and are applied thermally onto the bottom side.
The choice for silicone results in a first aspect from the fact that silicones have a larger friction coefficient in contact with a large scale of materials from which floors are typically made, and in particular with floors with an increased risk of slipping, such as parquet floor, laminate, vinyl, PVC and similar. In addition, silicone is also easy to process and is appropriate for binding with tissues comprising elastane and polyamide. By applying the silicone gripping elements thermally onto the bottom side, a strong binding is ensured, without the structure of the material of the compression sock itself having to be affected.

The foot part comprises at the upper side an opening at the distal end with respect to the leg part. Preferably, the opening extends over a lateral length of at least 50% of the width of the compression sock at the level of the opening, more preferably at least 75%, still more preferably at least 85% or even at least 95%. In this way, it is guaranteed that the complete toe zone (each toe) can easily be inspected. When the compression sock is worn, and a foot-shaped object is present, the opening is stretched laterally, till it extends over a minimal width of 50% of the foot, preferably to a minimal width of 75% or even 80, 85% of more of the foot. The longitudinal dimension of the opening for a compression sock that is worn, is typically limited to maximum 5 cm, preferably maximum 4 cm.
Hereby, it should be remarked that most preferably, the opening does not extend to the bottom side of the foot part. The reason is on the one hand to maximize the available surface for gripping elements, but on the other hand also to minimize the contact between the foot and the floor to have as little as possible dirt, dust, bacteria and similar come into contact with the foot. This is very important as the person wearing such a compression sock often already has a weakened system, and thus exposure to pathogenic elements should be avoided. Finally, the applicant remarks that it is advantageous not to have the opening at the most distal position (but in practice, at the beginning of the toes, for example the first and/or second toe pasterns) in order to have the toe ends covered in this way. In this way, the extreme extremities, the toes, of the body are better protected against the cold.

Such an inspection opening is in particular useful for evaluating the toes of the user for timely detection of possible problems (for example, too high compression, too low, or underlying problems of the user itself). Especially with (bedridden) patients in a hospital or at home, this saves the effort to put off the compression socks and to put them on again to check the condition of the foot, and it thus also saves a lot of time for a doctor or a nurse.

Preferably, the opening is defined by an elastic band. By a further attachment of the band, the laterally stretched shape of the opening is further ensured when wearing the sock.

In a preferred embodiment, the leg part is open at a distal end with respect to the foot part, and the leg part is provided at the distal open end with an inward directed gripping edge, in which the gripping edge comprises a plurality of inward directed leg gripping elements, the leg gripping elements are adapted for a better grip onto a piece of the leg of the user by increasing the friction coefficient between the gripping edge and the leg. The leg gripping elements are preferably substantially sphere-shaped. The leg gripping elements are preferably in rows parallel to the edge of the zoom, with the subsequent rows in a staggered pattern.

Preferably, the leg gripping elements have a maximal diameter of about 0.5 cm, more preferably maximal about 0.4 cm or even about 0.3 cm.

To avoid that the compression socks come down, they are provided with a specific gripping edge at the upper end. This upper edge is adapted for gripping onto the leg of the wearer, and it therefore has leg gripping elements. Preferably, they are smaller than the gripping elements and they are provided with a stronger (deeper) relief. Moreover, they are more preferably positioned close to each other. These aspects are all provided for improving the grip onto the leg of the wearer.

In a preferred embodiment, the leg gripping elements are located in an inward directed zoom around the leg part at the open end, in which the zoom has a minimal width of 1 cm, preferably, in which the leg gripping elements comprise silicone.

The edge should preferably have a minimal width of 1 cm and extend around the leg of the user to keep up the sock. The coming down of the compression sock can lead to dangerous situations and should thus definitely be avoided.

In a preferred embodiment, the gripping elements have a minimal thickness of about 0.15 mm. Preferably, they have a minimal thickness of about 0.2 mm, and more preferably a minimal thickness of about 0.25 mm.

Preferably, the gripping elements have a maximal thickness of about 0.6 mm, more preferably maximum about 0.5 mm, still more preferably about 0.4 mm and still more preferably maximum about 0.3 mm.

The applicant has noted that the provision of a certain relief onto the gripping elements at the bottom side of the foot part increases the friction coefficient. More in particular, a thickness of minimum 0.15 mm was already sufficient for causing a noticeable increase in friction. This further also ensures that the gripping elements can grip sufficiently strongly on a large number of kinds of floors, even if they would be particularly smooth.

In a preferred embodiment, the foot part and/or the leg part comprises at least 70% of polyamide and at least 10% of elastane. The percentage of polyamide is preferably at least 71% or at least 72%, 73%, 74%, 75%, 76%, 77%, 78%, 79%, 80%, 81%, 82% or more. The percentage of elastane is preferably at least 71% or at least 711%, 12%, 13%, 14%, 15%, 16%, 17%, 18% or more. Most preferably, it comprises 82% of polyamide and/or at least 18% of elastane.

The chosen substances in said proportions ensure a strongly elastic and robust compression sock that is capable of exerting the appropriate pressure onto the leg and/or foot of the wearer, and thus preventing blood clots from forming.

In a preferred embodiment, the gripping elements are essentially circular.
The applicant has noted that this shape with respect to the surface of the gripping elements is optimal to increase the friction coefficient (in which preferably, surfaces are specifically meant such as parquet floor, laminate, vinyl, PVC and similar).

In an alternative embodiment, the gripping elements have a shape from the following list: essentially triangular, essentially square, essentially rectangular, essentially elliptic, essentially S-shaped, essentially cross-shaped.
The alternative embodiments can moreover optionally be combined with each other and/or with circular gripping elements.

In a preferred embodiment, the leg part comprises an enlarged calf zone, positioned for and appropriate for receiving a calf of the user, and the leg part comprises a narrowed upper ankle zone, positioned for and appropriate for receiving a part of the leg above the ankle of the user, and in which the leg part comprises a material with, at a larger part of the leg, more stitches than at a smaller part of the leg for a more uniform elasticity and compression.

In a preferred embodiment, the leg part of the compressions socks has a length comprised between 11 cm and 80 cm, depending on amongst other things the wearer, as well as the choice to have the compression sock extend till under the knee, till (just) above the knee or till the thigh.

In a preferred embodiment, the material of the foot part and/or the leg part is a hypoallergic material.

In the following, the invention will be described by means of non-limiting examples illustrating the invention, and not meant to be interpreted as limiting the scope of the invention.

### EXAMPLES

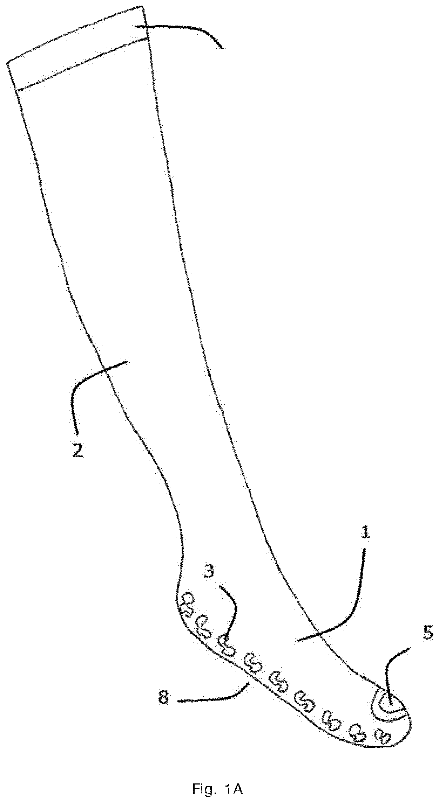

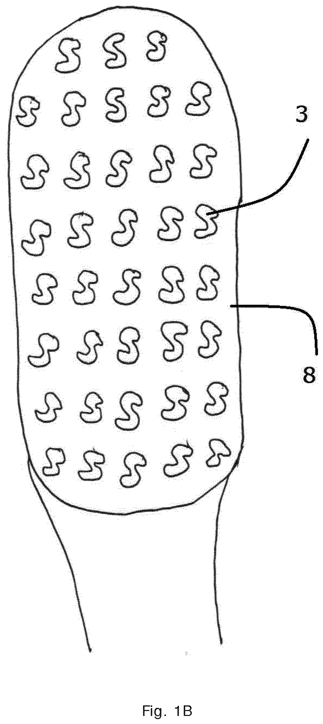

In Figure 1A-B, a first possible embodiment is shown, in which the compression sock is visible with a foot part (1) and a leg part (2), and further comprising a heel part and inspection opening (5) at the toe end. At the bottom side (8), a plurality of approximately S-shaped gripping elements (3) is visible, extending from the toe end to the heel part. At the end of the leg part (2) up to the cross of the wearer, a gripping edge (A) can optionally be provided.

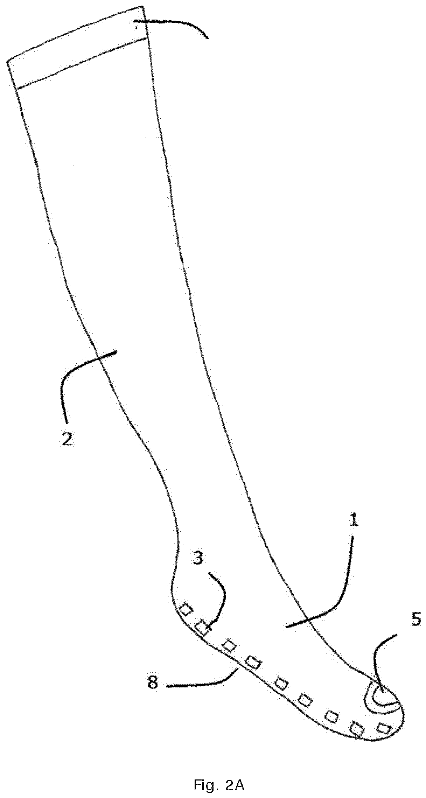

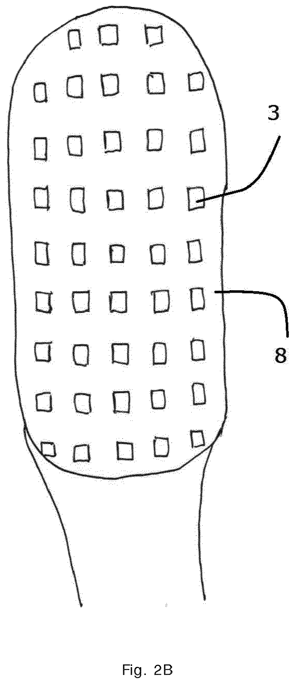

In Figure 2A-B, a second possible embodiment is shown with rectangular gripping elements (3).

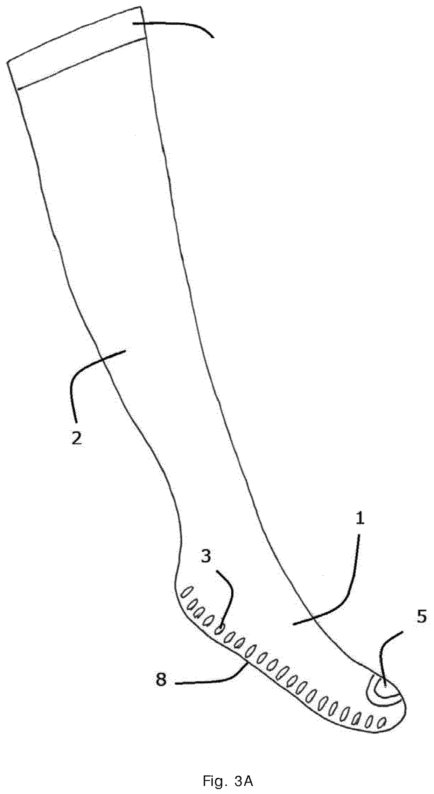

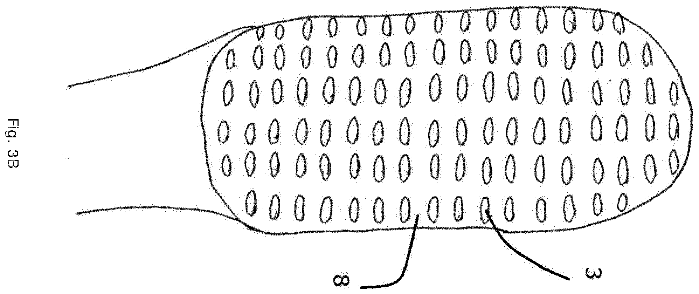

In Figure 3A-B, a second possible embodiment is shown with elliptic gripping elements (3), with the long axis of the ellipse perpendicular to the longitudinal axis of the foot part.

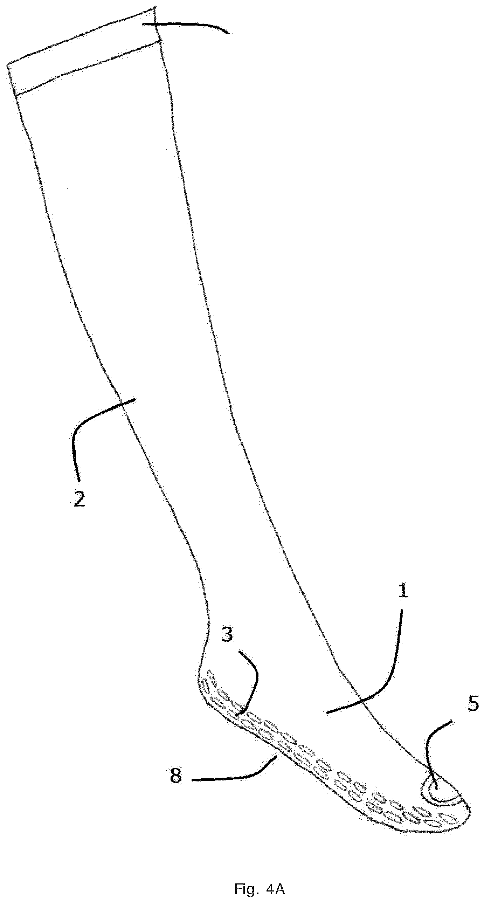

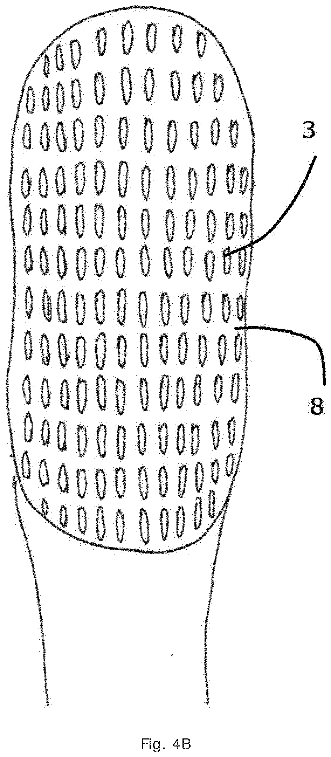

In Figure 4A-B, a second possible embodiment is shown with elliptic gripping elements (3), with the long axis of the ellipse parallel to the longitudinal axis of the foot part.

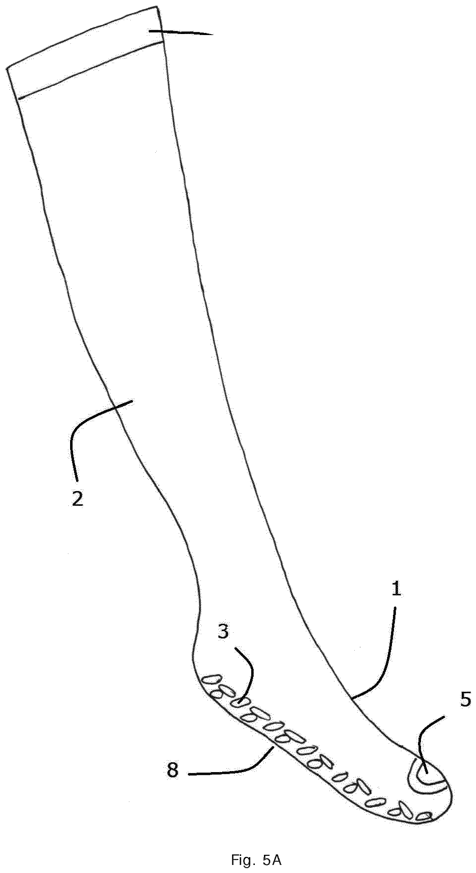

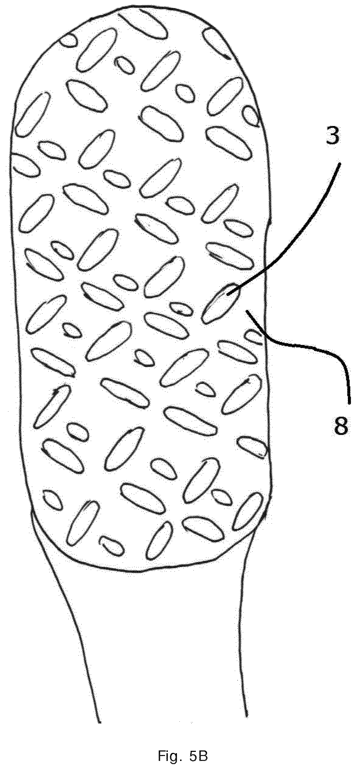

In Figure 5A-B, a second possible embodiment is shown with several elliptic gripping elements (3) with varying size.

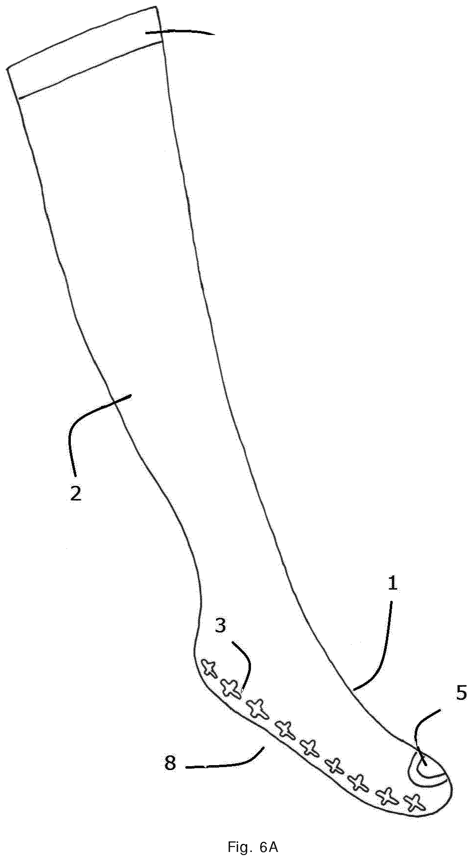

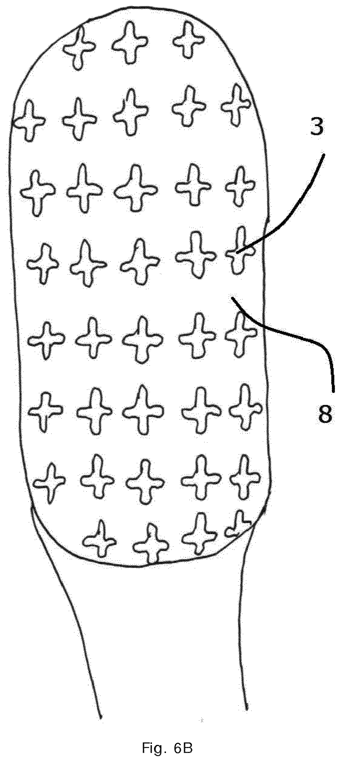

In Figure 6A-B, a second possible embodiment is shown with cross-shaped gripping elements (3).

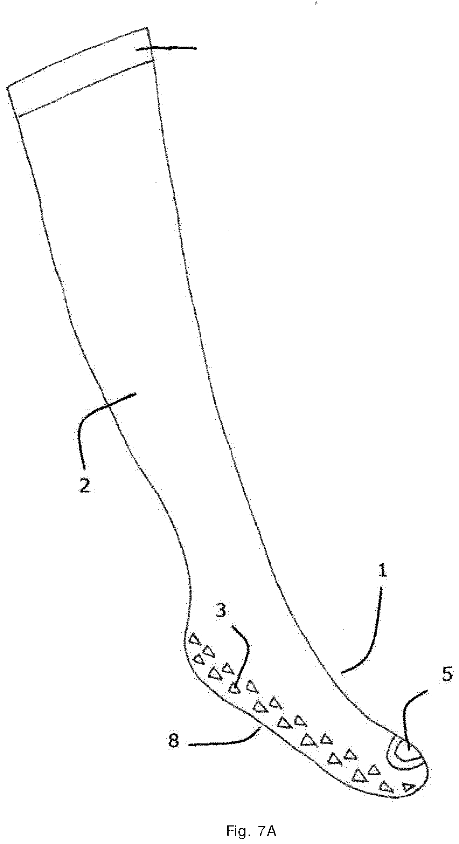

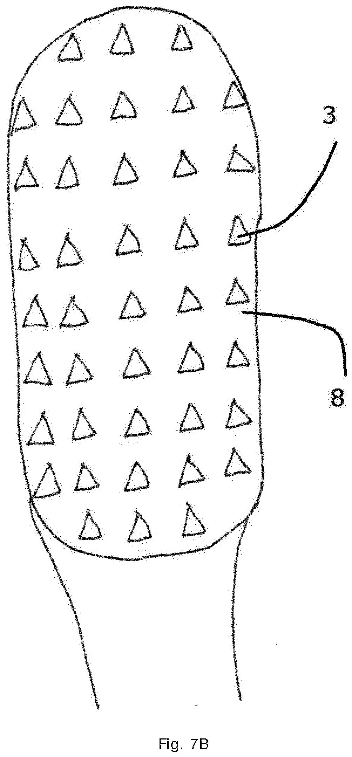

In Figure 7A-B, a second possible embodiment is shown with triangular gripping elements (3).

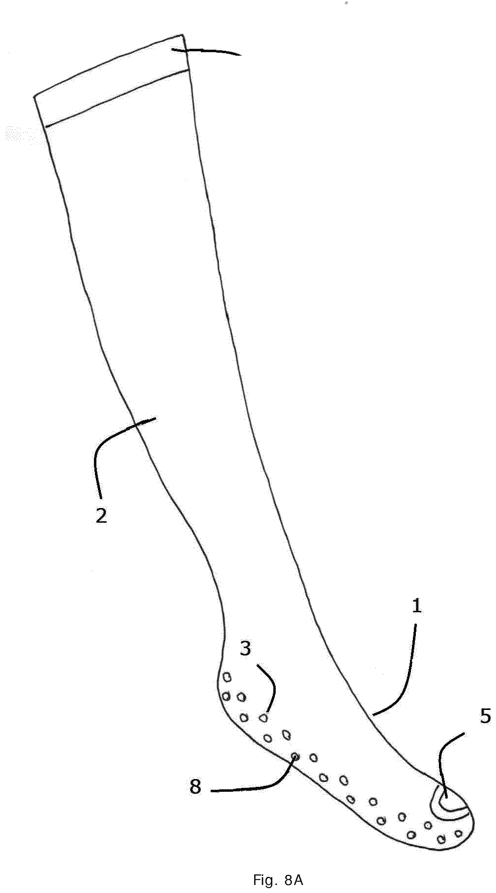

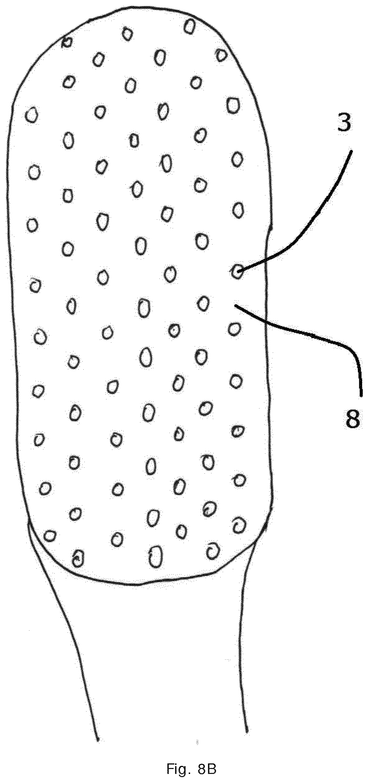

In Figure 8A-B, a second possible embodiment is shown with circular gripping elements (3).

It will be understood that the present invention is not limited to the embodiments described above and that some adjustments or changes can be added to the described examples without changing the scope of the enclosed claims. For example, the present invention is described with reference to medical compression socks, but it will clear that the invention can also be applied to e.g. non-medical compression socks.

## Claims

1. A compression sock for preventing blood clots from forming, comprising an elastic foot part (1) that is appropriate for receiving a foot of a user, and an elastic leg part (2) that is appropriate for receiving at least a part of a leg of the user, the part of the leg at least comprising the ankle and knee, and optionally the thigh of the user, in which the foot part (1) comprises an external bottom side (8) that is appropriate for the contact of the foot with the floor, **characterized in that** the the foot part (1) at the upper side thereof comprises an opening (5) at the distal end of the foot part (1) with respect to the leg part (2), and in which the external bottom side (8) of the foot part (1) is provided with a plurality of gripping elements (3) that are adapted for increasing the friction coefficient between the bottom side (8) and the floor, in which the gripping elements (3) each have a minimal surface of approximately 0.2 cm² and a maximal surface of approximately 1 cm², preferably a minimal surface of approximately 0.225 cm² and/or a maximal surface of approximately 0.50 cm², more preferably a maximal surface of approximately 0.40 cm², still more preferably a maximal surface of 0.35 cm² and most preferably a maximal surface of 0.30 cm².

2. The compression sock of the previous claim 1, wherein the gripping elements (3) are adapted to be transversally stretchable with respect to a longitudinal axis of the foot part (1) of the compression sock at least up to 110%, preferably at least up to 120% of a transversal width of the non-stretched gripping elements (3).

3. The compression sock of any one of the previous claims 1 or 2, in which the leg part (2) comprises an enlarged calf zone, positioned for and appropriate for receiving a calf of the user, and the leg part (2) comprises a narrowed upper ankle zone, positioned for and appropriate for receiving a part of the leg above the ankle of the user, and in which the leg part (2) comprises a woven material, wherein the woven material at the level of and around the calf zone comprises more stitches compared to the woven material at the level of and around the upper ankle zone.

4. The compression sock of any one of the previous claims 1 to 3, in which the gripping elements (3) have a longest dimension at the external bottom side of maximal about 0.75 cm, and preferably of maximal about 0.65 cm.

5. The compression sock of any one of the previous claims 1 to 4, in which the leg part (2) is adapted for exerting a pressure on the leg of the user, in which the pressure that is exerted by the leg part (2) in a zone closer to the foot part (1) is higher than the pressure that is exerted by the leg part (2) in a zone further from the foot part (1), preferably in which the pressure at the level of the ankle of the user is about 18 - 20 mmHg, and at the level of the knee of the user about 12 - 14 mmHg, and optionally in which the pressure at the level of the thigh of the user is about 6 - 8 mmHg.

6. The compression sock of any one of the previous claims 1 to 5, in which the foot part (1) comprises a specific heel part, in which the heel part is preformed and is appropriate for gripping and holding in position of the heel of the user, in which the foot part (1) comprises a specific toe part, in which the toe part is preformed anatomically and is appropriate for receiving toes of the user, **characterized in that** the gripping elements (3) extend at the bottom side at least from the heel part up to the toe part.

7. The compression sock of any one of the previous claims 1 to 6, in which the gripping elements (3) protrude from the external bottom side (8), in which the gripping elements (3) comprise silicone, and in which the gripping elements (3) are applied thermally onto the bottom side.

8. The compression sock of any one of the previous claims 1 to 7, in which the leg part (2) is open at a distal end with respect to the foot part (1), and is provided at the distal open end with an inward directed gripping edge, in which the gripping edge comprises a plurality of inward directed leg gripping elements, the leg gripping elements being adapted for a better grip onto a piece of the leg of the user by increasing the friction coefficient between the gripping edge and the leg.

9. The compression sock of the previous claim 8, in which the leg gripping elements are located in an inward directed seam around the leg part (2) at the open end, in which the seam has a minimal width of 1 cm, preferably, in which the leg gripping elements comprise silicone.

10. The compression sock of any one of the previous claims 1 to 9, in which the gripping elements (3) have a minimal thickness of about 0.15 mm, preferably a minimal thickness of about 0.2 mm, and still more preferably a minimal thickness of about 0.25 mm.

11. The compression sock of any one of the previous claims 1 to 10, in which the foot part (1) and/or the leg part (2) are composed of at least 70%, preferably at least 75% of polyamide and of at least 10% of elastane.

12. The compression sock of any one of the previous claims 1 to 11, in which the gripping elements (3) are essentially circular.

13. The compression sock of any one of the previous claims 1 to 11, in which the gripping elements (3) have a shape from the following list: essentially triangular, essentially square, essentially rectangular, essentially elliptic, essentially S-shaped, essentially cross-shaped.

14. The compression sock of any one of the previous claims 1 to 11, in which the leg part (2) comprises an enlarged calf zone, positioned for and appropriate for receiving a calf of the user, and the leg part (2) comprises a narrowed upper ankle zone, positioned for and appropriate for receiving a part of the leg above the ankle of the user, and in which the leg part (2) comprises a material with, at a larger part of the leg, more stitches than at a smaller part of the leg for a more uniform elasticity and compression.

15. Kit of 2 of more compressions socks of any one of the previous claims 1 to 14, in which at least one of the compression socks is appropriate for a right foot, and at least one of the compression socks is appropriate for a left foot.

## Patentansprüche

1. Kompressionsstrumpf zum Verhindern der Bildung von Blutgerinnseln, umfassend einen elastischen Fußteil (1), der zum Aufnehmen eines Fußes eines Benutzers geeignet ist, und einen elastischen Beinteil (2), der zum Aufnehmen zumindest eines Teils eines Beins des Benutzers geeignet ist, wobei der Teil des Beins zumindest das Fußgelenk und das Knie umfasst und optional den Oberschenkel des Benutzers, wobei der Fußteil (1) eine äußere Unterseite (8) umfasst, die für den Kontakt des Fußes mit dem Boden geeignet ist, **dadurch gekennzeichnet, dass** der Fußteil (1) an seiner Oberseite eine Öffnung (5) am distalen Ende des Fußteils (1) in Bezug auf den Beinteil (2) umfasst, und wobei die äußere Unterseite (8) des Fußteils (1) mit mehreren Gripp-Elementen (3) versehen ist, die zum Erhöhen des Reibungskoeffizienten zwischen der Unterseite (8) und dem Boden eingerichtet sind, wobei die Gripp-Elemente (3) jeweils eine Mindestoberfläche von etwa 0,2 cm² und eine Maximaloberfläche von etwa 1 cm² aufweisen, vorzugsweise eine Mindestoberfläche von etwa 0,225 cm² und/oder eine Maximaloberfläche von etwa 0,50 cm², bevorzugter eine Maximaloberfläche von etwa 0,40 cm², noch bevorzugter eine Maximaloberfläche von 0,35 cm² und am meisten bevorzugt eine Maximaloberfläche von 0,30 cm².

2. Kompressionsstrumpf nach dem vorhergehenden Anspruch 1, wobei die Gripp-Elemente (3) dafür eingerichtet sind, in Bezug auf eine Längsachse des Fußteils (1) des Kompressionsstrumpfs in Querrichtung mindestens bis zu 110%, vorzugsweise mindestens bis zu 120%, einer Querbreite der ungedehnten Gripp-Elemente (3) dehnbar zu sein.

3. Kompressionsstrumpf nach einem der vorhergehenden Ansprüche 1 oder 2, wobei der Beinteil (2) eine vergrößerte Wadenzone umfasst, die zum Aufnehmen einer Wade des Benutzers positioniert und geeignet ist, und der Beinteil (2) eine verschmälerte obere Fußgelenkzone umfasst, die zum Aufnehmen eines Teils des Beins oberhalb des Fußgelenks des Benutzers positioniert und geeignet ist, und wobei der Beinteil (2) ein gewebtes Material umfasst, wobei das gewebte Material auf Höhe der und um die Wadenzone mehr Stiche im Vergleich zu dem gewebten Material auf Höhe der und um die obere Fußgelenkzone umfasst.

4. Kompressionsstrumpf nach einem der vorhergehenden Ansprüche 1 bis 3, wobei die Gripp-Elemente (3) die längste Abmessung an der äußeren Unterseite (8) von maximal ungefähr 0,75 cm und vorzugsweise von maximal ungefähr 0,65 cm aufweisen.

5. Kompressionsstrumpf nach einem der vorhergehenden Ansprüche 1 bis 4, wobei der Beinteil (2) dafür eingerichtet ist, einen Druck auf das Bein des Benutzers auszuüben, wobei der Druck, der durch den Beinteil (2) in einer Zone ausgeübt wird, die näher zum Fußteil (1) liegt, höher ist als der Druck, der durch den Beinteil (2) in einer Zone ausgeübt wird, die weiter von dem Fußteil (1) entfernt liegt, vorzugsweise wobei der Druck auf Höhe des Fußgelenks des Benutzers ungefähr 18 - 20 mmHg beträgt und auf Höhe des Knies des Benutzers ungefähr 12 - 14 mmHg, und optional wobei der Druck auf Höhe des Oberschenkels des Benutzers ungefähr 6 - 8 mmHg beträgt.

6. Kompressionsstrumpf nach einem der vorhergehenden Ansprüche 1 bis 5, wobei der Fußteil (1) einen speziellen Fersenteil umfasst, wobei der Fersenteil vorgeformt ist und zum Greifen und An-Position-Halten der Ferse des Benutzers geeignet ist, wobei der Fußteil (1) einen speziellen Zehenteil umfasst, wobei der Zehenteil anatomisch vorgeformt ist und zum Aufnehmen von Zehen des Benutzers geeignet ist, **dadurch gekennzeichnet, dass** sich die Gripp-Elemente (3) an der Unterseite (8) mindestens von dem Fersenteil bis zu dem Zehenteil erstrecken.

7. Kompressionsstrumpf nach einem der vorhergehenden Ansprüche 1 bis 6, wobei die Gripp-Elemente (3) von der äußeren Unterseite (8) hervorstehen, wobei die Gripp-Elemente (3) Silikon umfassen und wobei die Gripp-Elemente (3) thermisch auf die Unterseite (8) aufgebracht sind.

8. Kompressionsstrumpf nach einem der vorhergehenden Ansprüche 1 bis 7, wobei der Beinteil (2) an einem distalen Ende in Bezug auf den Fußteil (1) offen ist und an dem distalen offenen Ende mit einem nach innen gerichteten Gripp-Rand versehen ist, wobei der Gripp-Rand mehrere nach innen gerichtete Beingripp-Elemente (3) umfasst, wobei die Beingripp-Elemente (3) für einen besseren Gripp an einem Stück des Beins des Benutzers durch Erhöhen des Reibungskoeffizienten zwischen dem Gripp-Rand und dem Bein eingerichtet sind.

9. Kompressionsstrumpf nach dem vorhergehenden Anspruch 8, wobei die Beingripp-Elemente (3) in einer nach innen gerichteten Naht um den Beinteil (2) an dem offenen Ende angeordnet sind, wobei die Naht eine Mindestbreite von 1 cm aufweist, vorzugsweise wobei die Beingripp-Elemente (3) Silikon umfassen.

10. Kompressionsstrumpf einem der vorhergehenden Ansprüche 1 bis 9, wobei die Gripp-Elementen (3) eine Mindestdicke von ungefähr 0,15 mm aufweisen, vorzugsweise eine Mindestdicke von ungefähr 0,2 mm und noch bevorzugter eine Mindestdicke von ungefähr 0,25 mm.

11. Kompressionsstrumpf nach einem der vorhergehenden Ansprüche 1 bis 10, wobei der Fußteil (1) und/oder der Beinteil (2) zu mindestens 70 %, vorzugsweise zu mindestens 75 %, aus Polyamid und zu mindestens 10 % aus Elastan bestehen.

12. Kompressionsstrumpf nach einem der vorhergehenden Ansprüche 1 bis 11, wobei die Gripp-Elemente (3) im Wesentlichen rund sind.

13. Kompressionsstrumpf nach einem der vorhergehenden Ansprüche 1 bis 11, wobei die Gripp-Elemente (3) eine Form aus der folgenden Liste aufweisen: im Wesentlichen dreieckig, im Wesentlichen quadratisch, im Wesentlichen rechteckig, im Wesentlichen elliptisch, im Wesentlichen S-förmig, im Wesentlichen kreuzförmig.

14. Kompressionsstrumpf nach einem der vorhergehenden Ansprüche 1 bis 11, wobei der Beinteil (2) eine vergrößerte Wadenzone aufweist, die zum Aufnehmen einer Wade des Benutzers positioniert und geeignet ist, und der Beinteil (2) eine verschmälerte obere Fußgelenkzone umfasst, die zum Aufnehmen eines Teils des Beins oberhalb des Fußgelenks des Benutzers positioniert und geeignet ist, und wobei der Beinteil (2) zwecks einer gleichmäßigeren Elastizität und Kompression ein Material mit mehr Stichen an einem größeren Teil des Beins als an einem kleineren Teil des Beins umfasst.

15. Satz aus 2 von mehr Kompressionsstrümpfen nach einem der vorhergehenden Ansprüche 1 bis 14, wobei mindestens einer der Kompressionsstrümpfe für einen rechten Fuß geeignet ist und mindestens einer der Kompressionsstrümpfe für einen linken Fuß geeignet ist.

## Revendications

1. Bas de contention pour empêcher des caillots sanguins de se former, comprenant une partie de jambe élastique (1) qui est appropriée pour recevoir un pied d'un utilisateur, et une partie de jambe élastique (2) qui est appropriée pour recevoir au moins une partie d'une jambe de l'utilisateur, la partie de la jambe comprenant au moins la cheville et le genou, et optionnellement la cuisse de l'utilisateur, dans lequel la partie de pied (1) comprend un côté inférieur externe (8) qui est approprié pour le contact du pied avec le sol, **caractérisé en ce que** la partie de pied (1) au niveau du côté supérieur de celle-ci comprend une ouverture (5) au niveau de l'extrémité distale de la partie de pied (1) par rapport à la partie de jambe (2), et dans lequel le côté inférieur externe (8) de la partie de pied (1) est doté d'une pluralité d'éléments d'adhérence (3) qui sont adaptés pour augmenter le coefficient de frottement entre le côté inférieur (8) et le sol, dans lequel les éléments d'adhérence (3) ont chacun une surface minimale d'approximativement 0,2 cm² et une surface maximale d'approximativement 1 cm², de préférence une surface minimale d'approximativement 0,225 cm² et/ou une surface maximale d'approximativement 0,50 cm², de manière davantage préférée une surface maximale d'approximativement de 0,40 cm², de manière encore davantage préférée une surface maximale de 0,35 cm² et de manière préférée entre toutes une surface maximale de 0,30 cm².

2. Bas de contention selon la revendication précédente 1, dans lequel les éléments d'adhérence (3) sont adaptés pour être étirables transversalement par rapport à un axe longitudinal de la partie de pied (1) du bas de contention au moins jusqu'à 110 %, de préférence au moins jusqu'à 120 % d'une largeur transversale des éléments d'adhérence (3) non étirés.

3. Bas de contention selon l'une quelconque des revendications précédentes 1 ou 2, dans lequel la partie de jambe (2) comprend une zone de mollet élargie, positionnée pour et appropriée pour recevoir un mollet de l'utilisateur, et la partie de jambe (2) comprend une zone de cheville supérieure rétrécie, positionnée pour et appropriée pour recevoir une partie de la jambe au-dessus de la cheville de l'utilisateur, et dans lequel la partie de jambe (2) comprend un matériau tissé, dans lequel le matériau tissé au niveau de et autour de la zone de mollet comprend davantage de points de liage comparativement au matériau tissé au niveau de et autour de la zone de cheville supérieure.

4. Bas de contention selon l'une quelconque des revendications précédentes 1 à 3, dans lequel les éléments d'adhérence (3) ont une dimension la plus longue au niveau du côté inférieur externe (8) de maximum environ 0,75 cm, et de préférence de maximum environ 0,65 cm.

5. Bas de contention selon l'une quelconque des revendications précédentes 1 à 4, dans lequel la partie de jambe (2) est adaptée pour exercer une pression sur la jambe de l'utilisateur, dans lequel la pression qui est exercée par la partie de jambe (2) dans une zone plus proche de la partie de pied (1) est plus élevée que la pression qui est exercée par la partie de jambe (2) dans une zone plus éloignée de la partie de pied (1), de préférence dans lequel la pression au niveau de la cheville de l'utilisateur est d'environ 18 - 20 mmHg, et au niveau du genou de l'utilisateur d'environ 12 - 14 mmHg, et optionnellement dans lequel la pression au niveau de la cuisse de l'utilisateur est d'environ 6 - 8 mmHg.

6. Bas de contention selon l'une quelconque des revendications précédentes 1 à 5, dans lequel la partie de pied (1) comprend une partie de talon spécifique, dans lequel la partie de talon est préformée et est appropriée pour adhérer et se maintenir dans la position du talon de l'utilisateur, dans lequel la partie de pied (1) comprend une partie d'orteil spécifique, dans lequel la partie d'orteil est préformée anatomiquement et est appropriée pour recevoir les orteils de l'utilisateur, **caractérisé en ce que** les éléments d'adhérence (3) s'étendent au niveau du côté inférieur (8) au moins à partir de la partie de talon jusqu'à la partie d'orteil.

7. Bas de contention selon l'une quelconque des revendications précédentes 1 à 6, dans lequel les éléments d'adhérence (3) font saillie à partir du côté inférieur externe (8), dans lequel les éléments d'adhérence (3) comprennent de la silicone, et dans lequel les éléments d'adhérence (3) sont appliqués thermiquement sur le côté inférieur (8).

8. Bas de contention selon l'une quelconque des revendications précédentes 1 à 7, dans lequel la partie de jambe (2) est ouverte au niveau d'une extrémité distale par rapport à la partie de pied (1), et est dotée au niveau de l'extrémité ouverte distale d'un bord d'adhérence dirigé vers l'intérieur, dans lequel le bord d'adhérence comprend une pluralité d'éléments d'adhérence (3) de jambe dirigés vers l'intérieur, les éléments d'adhérence (3) de jambe étant adaptés pour une meilleure prise sur une région de la jambe de l'utilisateur par augmentation du coefficient de frottement entre le bord d'adhérence et la jambe.

9. Bas de contention selon la revendication précédente 8, dans lequel les éléments d'adhérence (3) de jambe sont situés dans une couture dirigée vers l'intérieur autour de la partie de jambe (2) au niveau de l'extrémité ouverte, dans lequel la couture a une largeur minimale de 1 cm, de préférence, dans lequel les éléments d'adhérence (3) de jambe comprennent de la silicone.

10. Bas de contention selon l'une quelconque des revendications précédentes 1 à 9, dans lequel les éléments d'adhérence (3) ont une épaisseur minimale d'environ 0,15 mm, de préférence une épaisseur minimale d'environ 0,2 mm, et de manière encore davantage préférée une épaisseur minimale d'environ 0,25 mm.

11. Bas de contention selon l'une quelconque des revendications précédentes 1 à 10, dans lequel la partie de pied (1) et/ou la partie de jambe (2) sont composées d'au moins 70 %, de préférence d'au moins 75 % de polyamide et d'au moins 10 % d'élastane.

12. Bas de contention selon l'une quelconque des revendications précédentes 1 à 11, dans lequel les éléments d'adhérence (3) sont essentiellement circulaires.

13. Bas de contention selon l'une quelconque des revendications précédentes 1 à 11, dans lequel les éléments d'adhérence (3) ont une forme parmi la liste suivante : essentiellement triangulaire, essentiellement carrée, essentiellement rectangulaire, essentiellement elliptique, essentiellement en S, essentiellement en croix.

14. Bas de contention selon l'une quelconque des revendications précédentes 1 à 11, dans lequel la partie de jambe (2) comprend une zone de mollet élargie, positionnée pour et appropriée pour recevoir un mollet de l'utilisateur, et la partie de jambe (2) comprend une zone de cheville supérieure rétrécie, positionnée pour et appropriée pour recevoir une partie de la jambe au-dessus de la cheville de l'utilisateur, et dans lequel la partie de jambe (2) comprend un matériau comportant, au niveau d'une partie plus grande de la jambe, davantage de points de liage qu'au niveau d'une partie plus petite de la jambe pour une élasticité et une compression plus uniformes.

15. Kit de 2 bas de contention ou plus selon l'une quelconque des revendications précédentes 1 à 14, dans lequel au moins l'un des bas de contention est approprié pour un pied droit, et au moins l'un des bas de contention est approprié pour un pied gauche.
